Europäisches Patentamt

**European Patent Office**

Office européen des brevets

(19)

(11) Publication number: **0 175 283**
A1

# EUROPEAN PATENT APPLICATION

(12)

(21) Application number: **85111530.3**

(22) Date of filing: **12.09.85**

(51) Int. Cl.⁴: **C 12 N 15/00,** C 07 H 21/04,
C 12 P 21/02
// A61K39/29 , C12R1:865

(30) Priority: **13.09.84 JP 193765/84**

(43) Date of publication of application: **26.03.86**
**Bulletin 86/13**

(84) Designated Contracting States: **AT BE CH DE FR GB IT**
**LI LU NL SE**

(71) Applicant: **Takeda Chemical Industries, Ltd., 27,**
**Doshomachi 2-chome Higashi-ku, Osaka-shi**
**Osaka, 541 (JP)**

(72) Inventor: **Kikuchi, Masakazu, 4-16,**
**Higashitokiwadai 7-chome Toyono-cho, Toyono-gun**
**Osaka 563-01 (JP)**
Inventor: **Fujisawa, Yukio,**
**31-104, 1 Mikagenakamachi 4-chome, Higashinada-ku**
**Kobe Hyogo 658 (JP)**

(74) Representative: **von Kreisler, Alek, Dipl.-Chem. et al,**
**Deichmannhaus am Hauptbahnhof, D-5000 Köln 1 (DE)**

(54) **Recombinant dna and use thereof.**

(57) HBsAg can be produced by cultivating yeast containing
a recombinant DNA wherein a DNA in which a sequence
complementary with the 3′ end of 18S ribosomal RNA is added upstream of a DNA coding for hepatitis B virus surface
antigen is bound just after a sequence complementary with
the 3′ end of 18S ribosomal RNA located at the 5′ non-coding
region of yeast repressible acid phosphatase gene.

HBsAg thus obtained can be used as a vaccine to pre-
vent HBV infection.

EP 0 175 283 A1

**0 175 283**

## Recombinant DNA and Use Thereof

This invention relates to a novel recombinant DNA and use thereof.

Hepatitis B is a disease caused by a virus which frequently outbreaks particularly in the tropical regions of Africa, Southeast Asia and the Far East, and also it is epidemically considered to be a cause of a chronic hepatitis, liver cirrhosis and hepatoma. The cause of the disease is hepatitis B virus (hereafter abbreviated and referred to as HBV), a sort of DNA virus. It is a globular particle of 42nm in diameter and called Dane particle after the name of its finder. There exist HBV surface antigens in the external layer of the particle. They are categorized, adr, adw, ayr, ayw, etc. depending on the different nature of antigen, and adw and adr are the types found in Japan.

The presence of small spherical and filamentous particles as well as Dane particles have been detected in the blood of hepatitis B patients and the presence of HBsAg similar to Dane particles have been confirmed with these particles. It is known about other viruses that the antibody against externally existing antigen of the virus keeps off the infection of the virus itself, and in case of HBV the production of anti-hepatitis B vaccine based on HBsAg is considerable. However, as infected with HBV are only human beings and chimpanzees, the trial to have HBV infect cultivated cells has not been successful yet. Therefore, HBsAg is only obtained from the blood of infected patients. The quantity of microparticles thus obtained is just enough to meet demand for material for a test but not that for vaccine production at all.

The current progress of molecular biology has made it possible to introduce DNA coding for a foreign protein into a strain of microorganism to be transformed. If the structural gene for HBsAg (hereafter referred to as HBsAg gene) were expressed inside the strain of microorganism, a large quantity of HBsAg free from HBV infection would be produced, which would pave the way for materialiaing the hepatitis B vaccine.

Regarding ayw which is one of four types of HBsAg presently found i.e. adw, adr, ayw, ayr and is frequently found in Europe and America, the existing site of HBsAg gene and its sequence have been determined [Galibert, F. et al., Nature, 281, 646(1979); Charnay, P. et al. Nucleic Acids Res., 7. 335(1979)], and a report has been made on its expression as hybrid proteins inside Escherichia coli [Charnay, P. et al., Nature, 286, 393(1980); Edman, J. C. et al. Nature, 291, 503(1981)]. On the other hand, regarding adw and adr which are frequently found in Japan, some of the present inventors were successful in forming DNA containing adw type HBsAg gene. They have determined the DNA sequence of the gene thus formed and the site on genome, and yet paved the way for a large quantity of HBsAg production by means of cultivating a transformant of gene transformed with the recombinant DNA. (Japanese Patent Provisional Publication No. 194897/83; No. 201796/83; and No.

74985/84).

Though the expression system using yeast as a host organism is recently occupying a broad attention, the first experiment of forming HBsAg was carried out by Velenzuela, P. et al. [Nature, $\underline{298}$, 347(1982)] by using a transformant of S. cerevisiae. Namely, it is reported that $adw_2$ type HBsAg gene was joined to the downstream of the promoter located at 5' non-coding region of yeast alcohol dehydrogenase gene, and this gene was expressed inside the yeast to produce 10 to 25μg of HBsAg particles per liter of culture medium. As these HBsAg particles have a particle structure having properties similar to the 22 nm particles found in human serum, the usefulness of yeast as a host organism has been verified. Later Miyanohara, A. et al. [Proc. Natl. Acad. Sci. USA, $\underline{80}$, 1(1983)] reported that adr type HBsAg gene was expressed with use of the promoter located at 5' non-coding region of the repressible acid phosphatase gene (PHO5) of yeast to produce 340μg of HBsAg particles per liter culture medium. Hitzeman, R. A. et al. [Nucleic Acids Res, $\underline{11}$, 2745(1983)] admitted that an unknown type of HBsAg was expressed with use of the promoter located at yeast 3-phosphoglyceric acid kinase gene 5' non-coding region to produce 50μg of HBsAg per liter culture medium. Also Murray, K. et al. [The EMBO Journal $\underline{3}$, 645(1984)] reported on formation of HBsAg equivalent to less than 0.1 percent of cellular protein as the result of the expression of adyw type HBsAg by using PHO5 promoter.

Yield of HBsAg by the conventional method is so small that a greater improvement of the yield is considered to be necessary.

For the start of protein synthesis by prokaryote it is known that the existence of a sequence complementary with the 3'end of 16S ribosomal RNA [Shine - Dalgarno sequence; Nature 254, 34(1975)] at the leader sequence of 5' end of mRNA is playing an important role. On the other hand, no sequence clearly complementary with the 3' end of 18S ribosomal RNA(18S rRNA) has been confirmed at the 5' non-coding region of eukaryote. Therefore, in order to improve the production of HBsAg by the transformants of yeast, we have succeeded greatly in improving the efficiency of synthesizing proteins by way of building up an artificial sequence which is highly complementary with the 3' end of 18S rRNA. That is, though there exists in the -21 to -30 region of PHO5 promoter a complementary sequence at the 3' end of 18S rRNA, we built up an HBsAg gene expression plasmid joined with another complementary sequence to the downstream of the region. As a result of this, it has been recognized that the yeast into which this novel plasmid has been introduced would produce a substantial quantity of HBsAg highly exceeding the yield of HBsAg production up to now. This knowledge has contributed to accomplish the present invention.

The meanings of the symbols used both in the detaile application and the figures is shown on Table 1.

TABLE 1

| DNA | : | Deoxyribonucleic acid |
| A | : | Adenine |
| T | : | Thymine |
| G | : | Guanine |
| C | : | Cytosine |
| U | : | Uracil |
| $m_2^6A$ | : | $N^6, N^6$ – Dimethyl Adenine |
| RNA | : | Ribonucleic acid |
| rRNA | : | Ribosomal RNA |
| dATP | : | Deoxyadenosine triphosphate |
| dTTP | : | Deoxythymidine triphosphate |
| dGTP | : | Deoxyguanosine triphosphate |
| dCTP | : | Deoxycytidine triphosphate |
| ATP | : | Adenosine triphosphate |
| EDTA | : | Ethylenediaminetetraacetic acid |
| SDS | : | Sodium dodecyl sulfate |
| Gly | : | Glycine |
| Ala | : | Alanine |
| Val | : | Valine |
| Leu | : | Leucine |
| Ile | : | Isoleucine |
| Ser | : | Serine |
| Thr | : | Threonine |
| Cys | : | Cysteine |

| Met | : | Methionine |
| Glu | : | Glutamic acid |
| Asp | : | Aspartic acid |
| Lys | : | Lysine |
| Arg | : | Arginine |
| His | : | Histidine |
| Phe | : | Phenylalanine |
| Tyr | : | Tyrosine |
| Trp | : | Tryptophan |
| Pro | : | Proline |
| Asn | : | Asparagine |
| Gln | : | Glutamine |
| $Ap^r$ | : | Ampicillin Resistance |
| arsl | : | Autonomous replication sequence 1 |
| IR | : | inverted repeat |

### A brief description of drawings

Figure 1 shows DNA (the lower row) coding for adw type hepatitis B virus surface antigen and its amino acid sequence (the upper row).

Figure 2 shows a scheme for constructing the plasmid pPHO17 and the symbols E, S, B, H, and X represent EcoRI, SalI BamHI, HindIII and XhoI respectively.

Figure 3 shows a scheme for constructing the plasmid pPHO17-58 and the symbols E, B, X and H represent EcoRI, BamHI, XhoI and Hind III respectively.

Figures 4(1), (2) and (3) show the promoter region

**0 175 283**

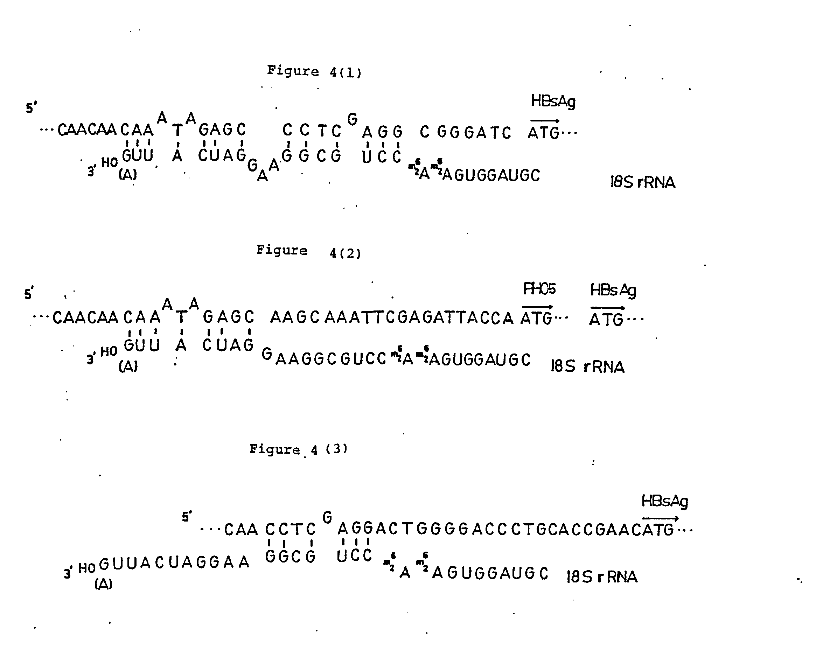

and the sequence of binding site of DNA coding for HBsAg (the upper row) and the sequence of the 3' end of 18S rRNA(the lower row). Figure 4(1) shows the sequences of the present invention and Figures 4(2) and (3) show the sequence of conventional techniques.

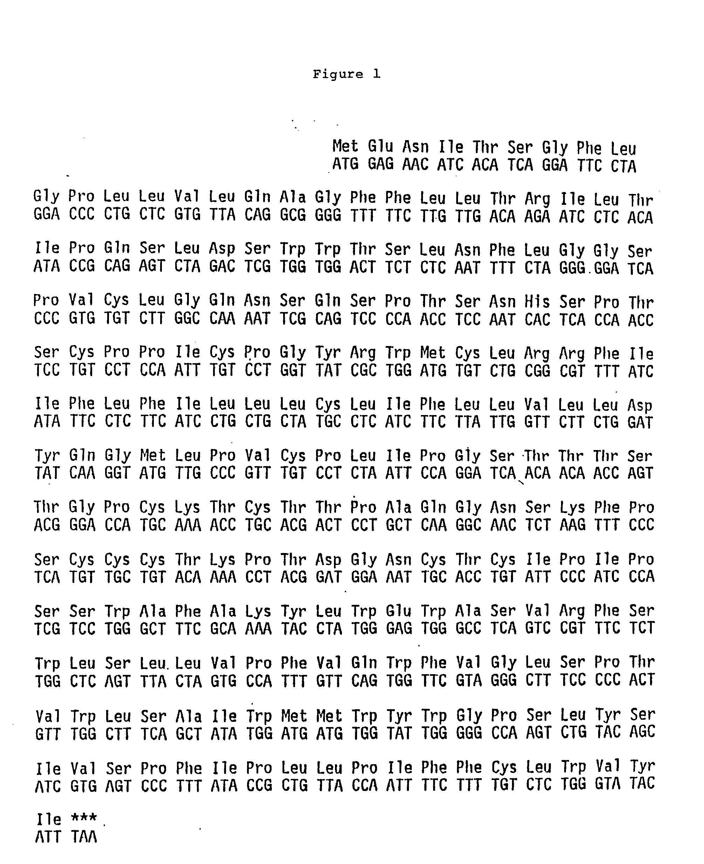

The present invention provides a recombinant DNA wherein a DNA in which a sequence complementary with the 3' end of 18S ribosomal RNA is added upstream of a DNA coding for hepatitis B virus surface antigen is bound just after a sequence complementary with the 3' end of 18S ribosomal RNA located at the 5' non-coding region of yeast repressible acid phosphatase gene, a method for producing the recombinant DNA, yeast containing the recombinant DNA, a method for producing the yeast transformant and a method for producing HBsAg. As regards the DNA encoding, for example, adw type hepatitis B virus surface antigen of all DNAs coding for hepatitis B virus surface antigen for the present invention, the DNA represented by the sequence shown in Fig. 1 is more preferable, although a DNA coding for the surface antigen of adw type hepatitis B virus can have any sequence so far as it codes for the surface antigen of adw type hepatitis B virus. This DNA can be either chemically synthesized or obtained from a DNA coding for HBV with enzymatic cleavage. The DNA, for example, is prepared by the following method.

931bp DNA fragment containing the whole sequence of DNA coding for HBsAg can be cleaved by digesting plamsid pHBV933 with restriction enzymes BamHI and HpaI. The plamsid pHBV933 incorporates DNA coding for 3.2 Kb of adw type HBV genome as described in Nucleic Acids Res, 11, 1747(1983). By partially digesting the DNA fragment with a restriction enzyme Sau3A, obtained is 809bp DNA fragment which is cleaved at the recognition site of Sau3A which exists immediately before the translation start codon ATG of DNA coding for adw type HBsAg. The DNA fragment thus obtained is made blunt at its ends by the action of DNA polymerase I large fragment to be combined with Bam HI linker d(CCGGATCCGG) and sub-cloned at BamHI site of plasmid pBR322 to obtain plasmid pHBs51. The plasmid thus obtained is digested with a restriction enzyme HpaII to obtain 815bp DNA fragment containing DNA coding for adw type HBsAg. The DNA fragment is made blunt at its ends by DNA polymerase I large fragment and combined with XhoI linker d(CCTCGAGG). Then preparation of 823bp DNA fragment with cohesive ends by a restriction enzyme XhoI would construct a sequence complementary with sequence 3' GUCC 5' located at the 3' end of 18S rRNA.

Subsequently, the expression vector of S. cerevisiae is constructed in accordance with the following method of Miyanohara, A. et al. (previously quoted). The method obtains plasmid pPHO 12 at the Bam HI-Sal I site of E. coli - S. cerevisiae shuttle vector - pSH 19 [Harashima, S. et al.; Mol. Cell. Biol., 4, 771(1984)] by inserting a 0.6 Kb DNA fragment obtained by the action of the restriction enzymes Bam HI and SalI on a plasmid pJA1 including PHO5 [Kramer, R. A. and Anderson, N.; Proc. Natl. Acad. Sci. USA, 77, 6541(1980)]. As this 0.6 Kb DNA fragment contains the promoter of PHO5 and a region coding for 27 amino acids at the N-terminal of PHO5, it is necessary to remove the latter region by the action of nuclease BAL-31. Then, after this plasmid pPHO 12 is cleaved by the action of the restriction enzyme Sal I, the coding region of the structural gene of PHO5 can be completely removed by the action of this nuclease and yet it is possible to construct a expression vector pPHO 17 which retains a sequence (CAAATAGAGC) located at -20 to -30 complementary with the 3' end of 18S rRNA.

By inserting 823bp DNA fragment containing DNA coding for the previously described adw type HBsAg into the Xho I site of the above mentioned expression vector pPHO 17, an expression plasmid pPHO 17-58 can be contructed.

HBsAg expression plasmids other than adw type (e.g. adr, ayr, ayw) can also be prepared in accordance with the above mentioned method.

By adopting these plasmids, leucine auxotrophic host yeast e.g. Saccharomyces cerevisiae AH22R⁻(a leu2 his4 can1 cir⁺pho80) [Miyanohara, A. et al. previsouly quoted], K33-7B (pho80-AH22, pho8-2) or K33-8D (pho80-AH22, pho8-2 trp1) is transformed by the known method [Hinnen, A. et al., Proc. Natl. Acad. Sci. USA, 75,1927(1978)] or other equivalent methods.

Besides, Saccharomyces cerevisiae AH22R⁻, which was deposited with the Institute of Fermentation under the designation of IFO-10134 and further it has been deposited with Fermentation Research Institute, Agency of Industrial Science and Technology, Ministry of International Trade and Industry, Japan (FRI) under the accession number of FERM P-7824 since September 4, ·1984, the deposit being converted to a deposit under the Budapest Treaty, has been stored at FRI under the accession number of FERM BP-804.

Thus obtained yeast transformant is cultured in the per se known culture medium. As for culture medium Burkholder minimum culture medium [Bostian, K. L. et al. Proc. Natl. Acad. Sci. USA, 77, 4505(1980)] is named as an example.

A yeast transformant is usually cultivated at 15°C to 43°C but favorably at 24°C to 37°C for 10 to 96 hours or for 24 to 72 hours, and airring and stirring can be added when necessary.

After cultivation, cells are collected by known method and suspended in the buffer solution, and then the cells are disrupted by means of Zymolyase [Seikagaku Kogyo Co., Ltd., Japan] or mechanical destruction such as glass beads. On this occasion HBsAg is effectively extracted by adding surface-active agent such as Triton X-100. To isolate HBsAg from the supernatant obtained by centrifugation of the culture, it is sufficient to apply the purification method of HBsAg particles derived from human serum.

Besides, the quantitative analysis of HBsAg can be done, according to the usual method, e. g. with use of AUSRIA II -125(Dainabot) or AUSZYME II (Dainabot).

When HBsAg is produced in accordance with the recombinant DNA of the present invention, the yield of HBsAg can be greatly increased compared with the production methods known up to now. Yet, HBsAg of the present invention will be used as an antiinfection vaccine for HBV.

Example

The reference examples and working examples shown below describe the present invention more concretely but the present invention will not be restricted to the scope of the description.

Reference Example 1

Construction of Expression vector containing yeast repressible acid phosphastase promotor

50 µg of E. Coli plasmids (pJAl)[Kramer, R. A. and Anderson, N., Proc. Natl. Acad. Sci. USA, 77, 6541 (1980)] containing 7.9 Kb DNA fragment which contains repressible acid phosphastase gene (PHO5) and constitutive acid phosphastase gene (PHO3) derived from the strain of Saccharomyces cerevisiae S288C were allowed to undergo the action of 20 unit restriction enzyme BamHI [Takara Shuzo, Japan] and 20 unit restriction enzyme SalI [Takara Shuzo, Japan] in 100 µl reaction mixture [10mM Tris-HCl (pH 8.0), 7mM $MgCl_2$, 100mM NaCl, 2mM 2-mercaptoethanol] at 37°C for 3 hours and then by using 1.0 % agarose (Sigma)slab gel they were electrophoresed in buffer solution [100mM Tris-HCl, 100mM boric acid, 2mM EDTA (pH 8.3)] for 2 hours at 140V. Following the electrophoresis, the gel fragment containing 0.63 Kb DNA fragment was enclosed in a dialysis tube and sunken in the buffer solution for electrophoresis to elute this DNA fragment out of the gel electrically [Dc Donell, M. W.

et al. J. Mol. Biol., 110, 119(1977)]. After the eluate in the dialysis tube was treated with phenol and then ethel, NaCl was added up to 0.2M which was followed by cold ethanol twice in quantity to precipitate the DNA at -20°C.

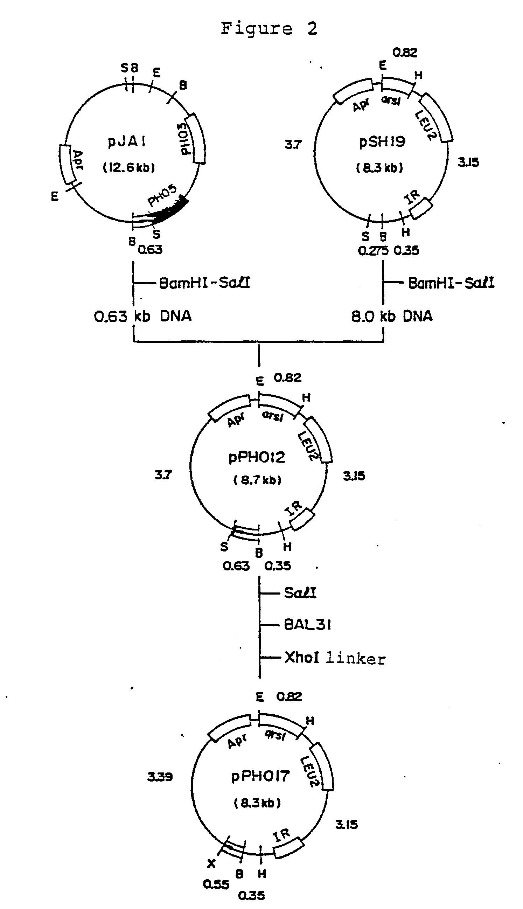

1 μg of plasmid pSH 19 was allowed to undergo the action of 2 unit restriction enzyme BamHI and 2 unit restriction enzyme SalI in 20 μl of reaction mixture [10mM Tris - HCl (pH 8.0), 7mM $MgCl_2$, 100mM NaCl, 2mM 2-mercaptoethanol] at 37°C for 2 hours, and then this reaction mixture was electrophoresed under the above-mentioned conditions using 0.8 % agarose slab gel. Following the electrophoresis 8.0 Kb DNA fragment was obtained from gel in accordance with the above-mentioned process, and the deproteinization with phenol was done, then DNA was precipitated with cold ethanol (refer to Fig. 2).

400ng of this 8.0 Kb DNA fragment and 200ng of foregoing 0.63 Kb DNA fragment were mixed, and in 20μl of reaction mixture [66mM Tris-HCl(pH 7.6), 6.6mM $MgCl_2$, 10mM dithiothreitol, 1mM ATP, 2 unit of T4 DNA ligase (Takara Shuzo Japan)] they were allowed to undergo the action for one night at 14°C and to be combined each other. Using this reaction mixture 294 strains of Escherichia coli (IFO-14171) were transformed in accordance with the foregoing treatment method by Cohen et al. By application of alkaline extraction method [Birnboim, H. C. and Doly, J., Nucleic Acids Res., 7, 1513(1979)] plasmid DNA was isolated from the transformant which had been selected by an ampicillin resistance marker.

Furthermore, the molecular weight and cleaveage pattern by restriction enzyme of the DNA were studied and plasmid pPHO12, in which 0.63 Kb DNA fragment isolated from pJA1 was inserted into the BamHI-SalI site of pSH19 (refer to Fig. 2).

3 μg of plasmid pPHO 12 DNA was allowed to undergo the action of 2 units of restriction enzyme SalI in 20μl of reaction mixture [10mM Tris-HCl(pH 7.5), 7mM MgCl$_2$, 175mM NaCl, 0.2M EDTA, 7mM 2-mercaptoethanol] at 37$^{\circ}$C. Following deproteinization with phenol, the DNA was precipitated with cold ethanol. 3 μg of this DNA was allowed to undergo the action of 12 units of BAL 31 nuclease (Bethesda Research Laboratories) in 50 μl of reaction mixture [20mM Tris-HCl (pH 8.1), 12mM CaCl$_2$, 12mM MgCl$_2$, 1mM EDTA] at 30$^{\circ}$C for 2 minutes. Following deproteinization with phenol, the DNA was precipitated in cold ethanol (refer to Fig. 2).

200 ng of Xho I linker d(CCTCGAGG) [New England BioLabs] was allowed to undergo the action of 3 units of T4 polynucleotide kinase [Takara Shuzo, Japan] in 50μl of reaction mixture [50mM Tris-HCl (pH 7.6), 10mM MgCl$_2$, 10mM 2-mercaptoethanol, 100μM ATP] at 37$^{\circ}$C for one hour and phosphorylated at the 5' end.

4 ng of the 5' end phosphorylated Xho I linker [5'-P-d(CCTCGAGG)] and 400 ng of foregoing pPHO 12 DNA which had been treated with BAL-31 were mixed and allowed to undergo the action of T4 DNA ligase under the conditions previously mentioned. Using the reaction mixture thus obtained 294 strains of Escherichia coli were transformed in accordance with the mthod of Cohen et al.  Plasmid DNA was isolated by the foregoing alkaline extraction method from the transformant which had been selected by an ampicillin resistance marker and then a plasmid pPHO 17 which gave a 0.55 Kb fragment upon double digestion with BamHI and Xho I was selected.  An analysis of the DNA sequence by the dideoxynucleotide synthesis chain termination method [Sanger, F. et al., Proc. Natl. Acad. Sci. USA, 74, 5463(1977)] has verified that the upstream 20 bp of PHO5 start codon ATG had been removed by the BAL-31 nuclease treatment (refer to Fig. 2).

Example

Construction of a recombinant DNA molecular which expresses adw type hepatitis B virus surface antigen gene and yeast transformation with DNA molecular thereof

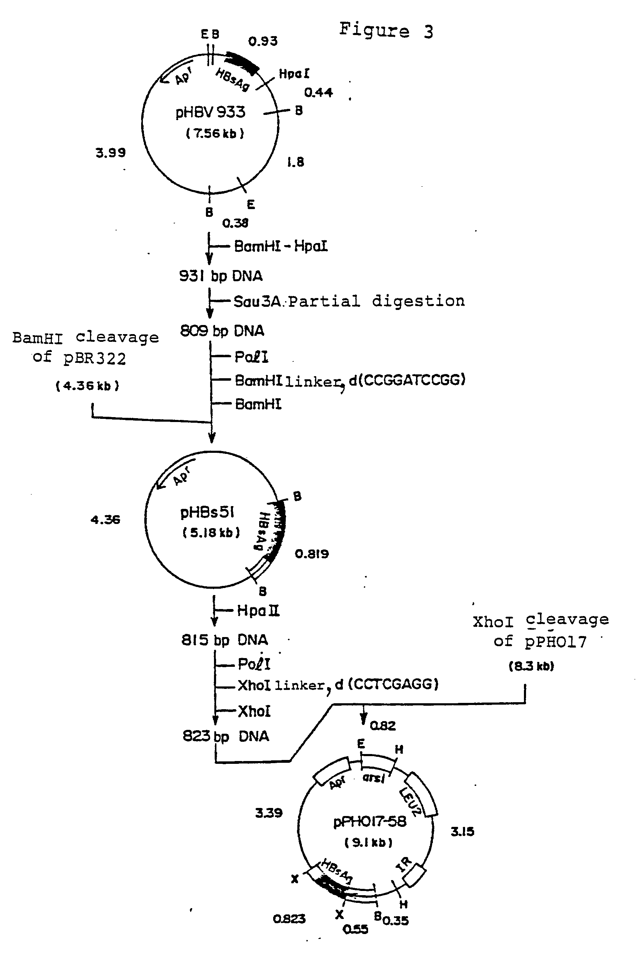

(1) 400 µg of plasmid pHBV 933 described in Nucleic Acids Res, 11, 1747(1983) was allowed to undergo the action of 50 units of restriction enzyme Bam HI and 20 units of restriction enzyme Hpa I (Takara Shuzo, Japan) in 800 µl of reaction mixture [10mM Tris-HCl (pH 7.5), 7mM $MgCl_2$, 100mM KCl, 7mM 2-mercaptoethanol] at $37^\circ$C for 24 hours, followed by an electrophoresis using 1.2 % agarose slab gel under the conditions described at Reference example 1.  After the electrophoresis 931bp DNA fragments were obtained from the gel in accordance with the method described at the said Reference Example 1 (refer to Fig. 3).

50 µg of 931 bp DNA fragment thus obtained was allowed to undergo the action of 50 units of restriction enzyme Sau 3A [Takara Shuzo, Japan] in 100 $\mu$l of reaction mixture [10mM Tris-HCl(pH 7.5), 7mM $MgCl_2$, 100mM NaCl] at $37^{\circ}C$ for 15 minutes, immediately followed by deproteinization with phenol. The reaction mixture was electrophoresed using 1.2% agarose slab gel under the conditions described at the foregoing Reference Example 1. After the electrophoresis 809bp DNA fragments were obtained from the gel in accordance with the method described at Reference example 1 (refer to Fig. 3).

1 $\mu$g of 809bp DNA thus obtained was allowed to undergo the action of 5 units of DNA polymerase I large fragment (New England BioLabs) in 30 $\mu$l of reaction fluid [40mM potassium phosphate buffer solution (pH 7.5), 6.6mM $MgCl_2$, 1mM 2-mercaptoethanol, 33$\mu$M dATP, 33$\mu$M dGTP, 33$\mu$M dTTP, 33$\mu$M dCTP] at $12^{\circ}C$ for 30 minutes and the cohesive ends of the DNA fragment were made blunt, followed by precipitation with cold ethanol. 500 ng of the DNA fragment thus obtained was mixed with 50 ng of phosphorylated BamHI linker [5'-p-d(CCGGATCCGG), Takara Shuzo, Japan] to be combined each other by the action of T4 DNA ligase under the conditions described at Reference Example 1 previously mentioned. 10 units of restriction enzyme BamHI were added to the reaction mixture at $37^{\circ}C$ for 3 hours to construct cohesive ends. After reaction, they were deproteinized with phenol and the samples were applied to Sepharose 4B (Pharmacia) column (0.25 X 25cm) which had been equilibrated with TEN buffer [10mM Tris-HCl (pH 8.0), 200mM NaCl, 1mM EDTA]. As the result, 819bp DNA fragments eluted around the void volume were collected and precipitated with cold ethanol. .

(2) 1 µg of plasmid pBR322 was treated with 2 units of restriction enzyme BamHI in 20 µl of reaction mixture [10mM Tris-HCl (pH 8.0), 7mM MgCl$_2$, 100mM NaCl, 2mM 2-mercaptoethanol] at 37$^O$C for 2 hours. After the reaction and deproteinization with phenol, DNA was precipitated in cold ethanol (BamHI-digested pBR322). 200 ng of this pBR 322 digested with BamHI and 200 ng of 819 bp DNA fragments previously mentioned were combined by the action of T4 DNA ligase under the conditions described in Reference Example 1. Using the reaction mixture, 294 strains of Escherichia coli were transformed and then separated was plasmid pHBs51 holding 819 bp DNA fragment inserted into its Bam HI site of plasmid pBR 322 in accordance with the method described at Reference Example 1 (refer to Fig. 3).

50 µg of plasmid pHBs 51 thus obtained wa treated with 20 units of restriction enzyme Hpa II [Takara Shuzo, Japan] in 100 µl of reaction mixture [10mM Tris-HCl (pH 7.4), 6mM KCl, 10mM MgCl$_2$, 1.0mM dithiothreitol] at 37$^O$C for 3 hours, and then the reaction mixture was electrophoresed in 1.5% agarose slab gel under the conditions described at Reference Example 1. After the electrophoresis, 815bp DNA fragments were obtained from the gel in accordance with the method described at Reference Example 1(refer to Fig. 3).

2 µg of 815 bp DNA fragment thus obtained was combined with phosphorylated Xho I linker as described at Reference Example 1 after its cohesive ends being made blunt with the action of DNA polymerase I large fragment under the conditions described above and then, cohesive ends were constructed with the treatment of Xho I. Fractions including 823bp DNA fragments were collected by use of Sepharose 4B column under the foregoing conditions and the DNA fragment was precipitated with cold ethanol.

(3) Expression vector pPHO 17 (1μg) described at Reference Example 1 was treated with 2 units of restriction enzyme Xho I in 20 μl of reaction mixture [10mM Tris-HCl(pH 7.5), 7mM $MgCl_2$, 100mM NaCl, 7mM 2-mercaptoethanol] at 37°C for 2 hours followed by deproteinization and precipitation of DNA with cold ethanol (Xho I-digested pPHO17).

200 ng of pPHO17 digested with XhoI thus obtained and 200 ng of 823bp DNA fragment previously mentioned were combined by the action of T4 DNA ligase under the conditions described at Reference Example 1. Using the reaction mixture thus obtained, 294 strains of Escherichia coli were transformed and a plasmid pPHO17-58 in which HBsAg gene contained in 823 bp DNA fragment was inserted in the same direction as PHO5 promoter was isolated in the manner described at Reference Example 1(refer to Fig. 3).

Yeast host Saccharomyces cerevisiae AH22R⁻ was transformed with this plasmid pPHO17-58 to obtain S. cerevisiae transformant (AH22R⁻/ pPHO17-58). The strain thus obtained (Saccharomyces cerevisiae AH22R⁻/pPHO17-58), which deposited with the Institute for Fermentation under No. IFO-10137 and also at Fermentation Research Institute, Agency of Industrial Science and Technology, Ministry of International Trade and Industry, Japan (FRI) with under the accession number of FERM P-7827 since September 4, 1984, the deposit being converted to a deposit under the Budapest Treaty, has been stored at FRI under the accession number of FERM BP-854.

Fig. 4 shows the sequence, at the junction site of promoter region and DNA coding for HBsAg, of the recombinant DNA [Fig. 4(1)] per this invention, the recombinant DNA [The EMBO Journal 3, 645(1984)] [Fig. 4(2)] with insertion of DNA coding for HBsAg into the downstream of PHO5 promoter and the recombinant DNA [Proc. Natl. Acad. Sci. USA, 80, 1(1983)] [Fig. 4(3)] lacking in the sequence complementary with the 3' end of 18S rRNA located at PHO5 promoter region. As it is clear from Fig. 4 the recombinant DNA of this invention is highly complementary with the sequence of the 3' end of 18S rRNA compared with the conventional one. Description of the yield of HBsAg per this invention will be made by the following example of production.

Example of Production

Expression of adw type HBsAg gene in yeast

Yeast transformant (Saccharomyces cerevisiae AH22R⁻/pPHO17-58) harboring adw type HBsAg gene expression plasmid obtained in accordance with Example was cultivated Burkholder and its low phosphoric acid culture medium at 30°C for 2 days and then, cells were collected and rinsed with isotonic sodium chloride solution. In accordance with the method by Miyanohara, A. et al. (previously mentioned), the cells were converted to spheroplast by the action of Zymolyase [Seikagaku Kogyo, Japan] and then, by adding 0.1% Triton X-100 to the spheroplast, HBsAg was extracted. The lysate was centrifuged at 15,000 rpm at room temperature for 15 minutes to give a supernatant. The HBsAg activity of the supernatant thus obtained was measured with Auszyme II [Dinabot]. The result was that the quantity of production of adw type HBsAg was 1.84 mg per liter.

CLAIMS

What is claimed is:

1. A recombinant DNA wherein a DNA in which a sequence complementary with the 3' end of 18S ribosomal RNA is added upstream of a DNA coding for hepatitis B virus surface antigen is bound just after a sequence complementary with the 3' end of 18S ribosomal RNA located at the 5' non-coding region of yeast repressible acid phosphatase gene.

2. The recombinant DNA according to claim 1, wherein the DNA coding for hepatitis B virus surface antigen is a DNA coding for adw type hepatitis B virus surface antigen.

3. The recombinant DNA according to claim 1, which is the expression plasmid pPHO17-58.

4. Yeast containing a recombinant DNA wherein a DNA in which a sequence complementary with the 3' end of 18S ribosomal RNA is added upstream of a DNA coding for hepatitis B virus surface antigen is bound just after a sequence complementary with the 3' end of 18S ribosomal RNA located at the 5' end non-coding region of yeast repressible acid phosphatase gene.

5. Yeast according to claim 4, wherein the host is leucine auxotrophic Saccharomyces cerevisiae.

6.   Yeast according to claim 4, wherein the host is Saccharomyces cerevisiae AH22R⁻.

7.   Yeast according to claim 4, which is Saccharomyces cerevisiae AH22R⁻/pPHO17-58.

8.   A method for producing hepatitis B virus surface antigen which comprises cultivating yeast containing a recombinant DNA wherein a DNA in which a sequence complementary with the 3' end of 18S ribosomal RNA is added upstream of a DNA coding for hepatitis B virus surface antigen is bound just after a sequence complementary with the 3' end of 18S ribosomal RNA located at the 5' non-coding region of yeast repressible acid phosphatase gene, accumulating hepatitis B virus surface antigen in the culture and recovering the antigen from the culture.

9.   A method for producing hepatitis B virus surface antigen according to claim 8, wherein the yeast is Saccharomyces cerevisiae AH22R⁻/pPHO17-58.

10.   A method for producing a recombinant DNA wherein a DNA in which a sequence complementary with the 3' end of 18S ribosomal RNA is added upstream of a DNA coding for hepatitis B virus surface antigen is bound just after a sequence complementary with the 3' end of 18S ribosomal RNA located at the 5' non-coding region of yeast repressible acid phosphatase gene, which comprises joining a sequence complementary with the 3' end of 18S ribosomal RNA to a DNA fragment coding for hepatitis B virus surface antigen, and inserting the thus obtained DNA into an expression vector which the region coding for repressible acid phosphatase is completely removed and which retains a sequence complementary with the 3' end of 18S ribosomal RNA located at -20 to -30 of a promoter region of the repressible acid phosphatase gene.

11. A method for producing yeast containing a recombinant DNA wherein a DNA in which a sequence complementary with the 3' end of 18S ribosomal RNA is added upstream of a DNA coding for hepatitis B virus surface antigen is bound just after a sequence complementary with the 3' end of 18S ribosomal RNA located at the 5' non-coding region of yeast repressible acid phosphatase gene, which comprises transforming yeast with the said recombinant DNA.

| | | | |
|---|---|---|---|

0 175 283

Figure 1

```
                                    Met Glu Asn Ile Thr Ser Gly Phe Leu
                                    ATG GAG AAC ATC ACA TCA GGA TTC CTA

Gly Pro Leu Leu Val Leu Gln Ala Gly Phe Phe Leu Leu Thr Arg Ile Leu Thr
GGA CCC CTG CTC GTG TTA CAG GCG GGG TTT TTC TTG TTG ACA AGA ATC CTC ACA

Ile Pro Gln Ser Leu Asp Ser Trp Trp Thr Ser Leu Asn Phe Leu Gly Gly Ser
ATA CCG CAG AGT CTA GAC TCG TGG TGG ACT TCT CTC AAT TTT CTA GGG GGA TCA

Pro Val Cys Leu Gly Gln Asn Ser Gln Ser Pro Thr Ser Asn His Ser Pro Thr
CCC GTG TGT CTT GGC CAA AAT TCG CAG TCC CCA ACC TCC AAT CAC TCA CCA ACC

Ser Cys Pro Pro Ile Cys Pro Gly Tyr Arg Trp Met Cys Leu Arg Arg Phe Ile
TCC TGT CCT CCA ATT TGT CCT GGT TAT CGC TGG ATG TGT CTG CGG CGT TTT ATC

Ile Phe Leu Phe Ile Leu Leu Leu Cys Leu Ile Phe Leu Leu Val Leu Leu Asp
ATA TTC CTC TTC ATC CTG CTG CTA TGC CTC ATC TTC TTA TTG GTT CTT CTG GAT

Tyr Gln Gly Met Leu Pro Val Cys Pro Leu Ile Pro Gly Ser Thr Thr Thr Ser
TAT CAA GGT ATG TTG CCC GTT TGT CCT CTA ATT CCA GGA TCA ACA ACA ACC AGT

Thr Gly Pro Cys Lys Thr Cys Thr Thr Pro Ala Gln Gly Asn Ser Lys Phe Pro
ACG GGA CCA TGC AAA ACC TGC ACG ACT CCT GCT CAA GGC AAC TCT AAG TTT CCC

Ser Cys Cys Cys Thr Lys Pro Thr Asp Gly Asn Cys Thr Cys Ile Pro Ile Pro
TCA TGT TGC TGT ACA AAA CCT ACG GAT GGA AAT TGC ACC TGT ATT CCC ATC CCA

Ser Ser Trp Ala Phe Ala Lys Tyr Leu Trp Glu Trp Ala Ser Val Arg Phe Ser
TCG TCC TGG GCT TTC GCA AAA TAC CTA TGG GAG TGG GCC TCA GTC CGT TTC TCT

Trp Leu Ser Leu Leu Val Pro Phe Val Gln Trp Phe Val Gly Leu Ser Pro Thr
TGG CTC AGT TTA CTA GTG CCA TTT GTT CAG TGG TTC GTA GGG CTT TCC CCC ACT

Val Trp Leu Ser Ala Ile Trp Met Met Trp Tyr Trp Gly Pro Ser Leu Tyr Ser
GTT TGG CTT TCA GCT ATA TGG ATG ATG TGG TAT TGG GGG CCA AGT CTG TAC AGC

Ile Val Ser Pro Phe Ile Pro Leu Leu Pro Ile Phe Phe Cys Leu Trp Val Tyr
ATC GTG AGT CCC TTT ATA CCG CTG TTA CCA ATT TTC TTT TGT CTC TGG GTA TAC

Ile ***
ATT TAA
```

Figure 2

Figure 3

5' ...CAACAACAA ᴬ ᵀ ᴬ GAGC   CCTC ᴳ AGG  C GGGATC ATG···  HBsAg

3' ᴴᴼ GUU ᴬ CUAG ᴳᴬ GGCG UCC m²₂Aᵐ²₂AGUGGAUGC  18S rRNA
      (A)

Figure 4(1)

5' ...CAACAA CAA ᴬ ᵀ ᴬ GAGC  AAGC AAATTCGAGATTACCA ATG···  FHO5   HBsAg ATG···

3' ᴴᴼ GUU ᴬ CUAG ᴳ AAGGCGUCC m²₂Aᵐ²₂AGUGGAUGC  18S rRNA
      (A)

Figure 4(2)

5' ...CAA CCTC ᴳ AGGACTGGGG ACCCTGCACCGGAACATG···  HBsAg

3' ᴴᴼ GUUACUAGGAA GGCG UCC ᴳ m²₂A ᵐ²₂AGUGGAUGC  18S rRNA
      (A)

Figure 4 (3)

| Category | Citation of document with indication, where appropriate, of relevant passages | Relevant to claim | CLASSIFICATION OF THE APPLICATION (Int. Cl 4) |
|---|---|---|---|
| | **DOCUMENTS CONSIDERED TO BE RELEVANT** | | EP 85111530.3 |
| A | EP - A2 - 0 105 149 (SCIENCE AND TECHNOLOGY AGENCY) <br> * Claims 1,7,18-20 * <br> -- | 1,5,6 | C 12 N 15/00 <br> C 07 H 21/04 <br> C 12 P 21/02 <br> // A 61 K 39/29 <br> C 12 R 1:865 |
| A | EP - A1 - 0 073 657 (GENENTECH) <br> * Claims 1,3,4,7,8 * <br> -- | 1,5,8 | |
| D,A | EP - A2 - 0 068 719 (TAKEDA) <br> * Claims 1-4 * <br> & JP-A2-58-201 796 <br> -- | 1,2,4 | |
| A | EP - A1 - 0 013 828 (BIOGEN) <br> * Claims 1,2,20,23 * <br> -- | 1,4 | |
| D,A | NATURE, vol. 298, no. 5872, 22 July 1982 (New York) <br><br> P. VALENZUELA et al. "Synthesis and assembly of hepatitis B virus surface antigen particles in yeast" pages 347-350 <br> ---- | | **TECHNICAL FIELDS SEARCHED (Int. Cl.4)** <br><br> C 12 N <br> C 07 H <br> C 12 P |

The present search report has been drawn up for all claims

| Place of search | Date of completion of the search | Examiner |
|---|---|---|
| VIENNA | 18-12-1985 | FARNIOK |